# EUROPEAN PATENT APPLICATION

(11) **EP 4 317 427 A1**
(43) Date of publication of application: **07.02.2024**
(21) Application number: 22734862.0
(22) Date of filing: 23.03.2022
(51) Int. Cl.: C12N 9/26, C12P 19/14

(54) **MODIFIED LEVANSUCRASE FOR THE OPTIMISED PRODUCTION OF FRUCTOOLIGOSACCHARIDES**

(30) Priority: 30.03.2021 CU 20210020
(71) Applicant: Centro de Ingeniería Genética y Biotecnología, La Habana 12100 (CU)
(72) Inventor: HERNÁNDEZ GARCÍA, Lázaro, La Habana 13500 (CU); QUINTERO DE LA OLIVA, Yamira, La Habana 17100 (CU); MARTÍNEZ PEÑA, Ana, Gabriela, La Habana 10700 (CU); MENÉNDEZ RODRÍGUEZ, Carmen, La Habana 12100 (CU); MUSACCHIO LASA, Alexis, Artemisa 32100 (CU); RAMÍREZ IBAÑEZ, Ricardo, La Habana 12100 (CU); CÉSPEDES HERNÁNDEZ, Odet, La Habana 12100 (CU); MARTÍNEZ GARCÍA, Duniesky, Sancti Spíritus 60200 (CU); PÉREZ CRUZ, Enrique, Rosendo, Sancti Spíritus 60200 (CU)
(74) Representative: V.O.
(86) International application number: PCT/CU2022/050003
(87) International publication number: WO 2022/207017

(57) **Abstract**

Modified levansucrase from *Gluconacetobacter diazotrophicus* (LsdA) comprising an amino acid sequence selected from the group of sequences composed of SEQ ID NO: 26 - SEQ ID NO: 34, as well as the genetically modified microorganism which produces the enzyme. This modified enzyme maintains high specific activity, converts sucrose into short-chain fructooligosaccharides of varied fructosyl linkages, and does not produce the polysaccharide levan. The invention also provides an enzyme preparation of the modified levansucrase; as well as the enzymatic process for conversion of sucrose into fructooligosaccharides using a modified levansucrase compriosing an amino acid sequence selected from the group of sequences composed of SEQ ID NO: 26 - SEQ ID NO: 34 or the genetically modified microorganism which produces the enzyme.

## Description

### Technical field

The present invention is related to the field of enzyme engineering and industrial biotechnology, in particular with the modification of the amino acid sequence of *Gluconacetobacter diazotrophicus* levansucrase (LsdA) to obtain modified variants with high specific activity and higher yield of fructooligosaccharides (FOS), based on the loss of the formation capacity of the polysaccharide levan. The invention has its main application in the establishment and improvement of enzymatic processes for the production of different types of short-chain FOS, soluble prebiotic fibers of wide commercial demand.

### Previous art

FOS are natural prebiotic fibers in high demand in the functional food market. The consumption of FOS favors the increase in the population of probiotic microorganisms (eg bifidobacteria and lactobacilli) in the colon, which brings associated benefits to the health of humans and animals (Singh y col., Appl. Biochem. Biotechnol. 184: 613-635, 2017). FOS are short-chain fructans, containing between 2 and 10 fructose units linked β-(2→1) (inulin type) or β-(2→6) (levan type).

Currently, only inulin-type FOS are marketed. Of these, 1-kestotriose [aglu(1,2)βfru(1,2)βfru] is the one with the highest commercial value, because it exerts an efficient prebiotic action in the proximal colon and its sweetening power is higher than that of the FOS with 3 or more fructose units (Tochio y col., Foods 7: 140, 2018).

Levan-type FOS have a high prebiotic action demonstrated in humans and animals, but they are not yet available on the market, so the establishment of a production process on an industrial scale that makes their commercialization economically feasible is of special interest (Porras-Dominguez y col., Carbohydrate Polymers 177: 40-48, 2017).

Currently, the commercial production of FOS from sucrose is carried out using β-fructofuranosidases (EC 3.2.1.26) or β-fructosyltransferases (EC 2.4.1.100) of fungal origin. Incubation of any of these enzymes at high concentrations (600-800 g/L) of sucrose (GF) produces 1-kestotriose (GF2), 1,1-kestotetraose (GF3), and 1,1,1-kestopentaose (GF4), whose sum reaches to represent 55-60% (weight/weight) of the total carbohydrates in the reaction mixture (Flores-Maltos y col., Critical Rev. Biotechnol. 36: 259-267, 2016).

In the commercial products Meioligo^{®} and Actilight^{®}, the ratio of GF2:GF3:GF4 is approximately 37:53:10. Another particularly attractive enzyme for the conversion of sucrose to 1-kestotriose is sucrose:sucrose 1-fructosyltransferase (1-SST, EC 2.4.1.99) from the plant *Schedonorus arundinaceus* produced in the yeast *Pichia pastoris* (Hernandez y col., J. Biotechnol. 266: 59-71, 2018). The recombinant enzyme, called Sal-SSTrec, is stable and converts sucrose into a mixture of GF2 and GF3 in a 9:1 ratio, with a total yield of FOS (55-60%, w/w). The recombinant enzyme Sal-SSTrec is not yet commercially available.

Fungal and plant fructosyltransferases have, as a common drawback, intrinsic exofructanase activity and hydrolyze their own FOS products, as the sucrose substrate is depleted (Hernández y col., J. Biotechnol. 266: 59-71, 2018). In the processes that use these enzymes for commercial purposes, it is necessary to stop the reaction when the yield of FOS reaches 50-60% (w/w) and sucrose still ranges between 15 and 25% (w/w) of the total composition. The remaining sucrose and the monosaccharides (glucose and fructose) present in the reaction mixture (FOS-55% syrup) are energy contaminants that must be eliminated in hypocaloric products intended for diabetics.

Currently, the industrial purification of FOS up to a degree of purity of 95% (w/w) is carried out using chromatography techniques, based on the molecular exclusion principle, where the removal of the disaccharide sucrose constitutes the main loss factor in the recovery of the trisaccharide 1-kestotriose. There is no report of an enzymatic procedure that allows the total conversion of the remaining sucrose in the reaction mixtures of eukaryotic fructosyltransferases, without leading to the hydrolysis of the FOS products. Levansucrase (EC 2.4.1.10) and inulosucrase (EC 2.4.1.9), secreted by certain bacteria, could be used in the production of different types of FOS from sucrose (Diez-Municio y col., Appl. Microbio.l Biotechnol. 100: 6251-6263, 2016; Kirtel y col., Appl. Microbiol. Biotechnol. 102: 9207-9220, 2018). The type of linkage, the degree of polymerization (DP) and the yield of FOS vary depending on the origin of the enzyme and the reaction conditions (Oner y col., Biotechnol. Advances 34: 827-844, 2016). In general, bacterial fructosyltransferases convert less than half of the initial sucrose content (50-70%, w/v) present in the reaction to short-chain FOS. The rest of the substrate is consumed via direct hydrolysis (invertase activity) and, to a large extent, by the processive polymerization of FOS products (polymerase activity). The formation of high molecular weight levan or inulin polysaccharides, in addition to decreasing the final yield of short-chain FOS, causes a notable increase in the viscosity of the reaction mixture, which complicates the biocatalyst operation process and the separation of the products by chromatography. For this reason, bacterial levansucrases and inulosucrases are considered unattractive candidates for the industrial production of FOS. (Oner y col., Biotechnol. Advances 34: 827-844, 2016).

*Gluconacetobacter diazotrophicus* levansucrase (named LsdA; GenBank: L41732.5), either recovered from the culture supernatant of the native bacterium (Hernández y col., Biochemical J. 309: 113-118, 1995), or recombinantly produced in yeast *P. pastoris* (Trujillo et al., Enzyme Microbial Technol. 28: 139-144, 2001), is particularly distinguished by the accumulation of the products 1-kestotriose and 1,1-kestotetraose [aglu(1,2)βfru(1,2)βfru(1,2)βfru] during the course of sucrose transformation. LsdA also synthesizes 6-kestotriose [aglu(1,2)βfru(6,2)βfru], a trisaccharide that acts as a direct precursor to the formation of the polysaccharide levan. This homopolymer reaches 10⁴ fructose units and is structured in β-(2→6) linked main chains, with β-(2→1) branch points. The formation of the polysaccharide levan occurs by a processive mechanism, where the enzyme remains attached to the fructosylated product, which favors its continuous elongation without mediating the release of intermediate oligofructans (Raga-Carbajal y col., Glycobiology 26: 377-385, 2016). In the absence of the sucrose substrate, LsdA exhibits exolevanase activity, but does not hydrolyze inulin-type fructans (Hernández et al., Biochemical J. 309: 113-118, 1995).

Currently, there is no modified fructosyltransferase of any origin that can be used, either as a single enzyme or a complementary enzyme, in a commercial production process of FOS with total conversion of the sucrose substrate. The successive elongation of the FOS products to form the polysaccharide levan is the main catalytic impediment presented by the enzyme LsdA for its industrial use in the direct production of FOS, or in the conversion of the remaining sucrose in the reaction mixtures of eukaryotic fructosyltransferases.

### Description of the invention

The present invention solves the aforementioned problems, by providing a modified *Gluconacetobacter diazotrophicus* levansucrase (LsdA) that is characterized by comprising an amino acid sequence selected from the group of sequences composed of SEQ ID NO: 26 - SEQ ID NO: 34. This modified variant of LsdA synthesizes short-chain FOS, which show variation in the type of fructosyl bond, and does not form the polysaccharide levan. Furthermore, it does not hydrolyze 1-kestotriose or 1,1-kestotetraose, products that tend to accumulate during the course of sucrose transformation.

The use of a chemical method of random mutagenesis, as an initial work tool, together with the massive screening by phenotype of the libraries of recombinant clones of *Escherichia coli,* allowed the identification of several amino acids of the active site of LsdA involved in the polymerization reaction (synthesis of the levan polymer). Several of these amino acids have structural equivalents in other bacterial fructosyltransferases, but their function remains unexplored. Of the amino acids identified, R141, H142, F274, N276 and R304 were selected (numbering referred to the sequence of the mature enzyme, that is, the first 30 amino acids that form the signal peptide are not considered) as targets for directed evolution work by saturation mutagenesis. By this way, libraries of LsdA variants containing all possible substitutions in these five amino acids, which directly or indirectly interact with the donor substrate and/or the acceptor substrate of the fructosyl residue, were constructed. The optimization of the change of the target amino acids allowed obtaining groups of modified LsdA of high specific activity, which do not produce the polysaccharide levan and which differ from each other in the yield and spectrum of the FOS products.

In the invention, 25 amino acids that are directly, or indirectly, involved in the formation of the polysaccharide levan (polymerization reaction) were identified in the LsdA sequence. To achieve this result, the construction of plasmid pALS200 expressing a chimeric gene encoding the precursor protein LsdA fused to a 6xHis tail under the constitutive expression *nptII* promoter was performed. The recognition and proteolytic processing of the LsdA signal peptide by the host *E*. *coli* allows the secretion of the mature protein (sequence devoid of the first 30 amino acids present in the precursor protein) to the cell periplasm, where exogenous sucrose penetrates and serves as substrate to the recombinant enzyme. The host bacterium containing active LsdA in the periplasmic space acquires the ability to utilize sucrose (Sac⁺ phenotype) and consequently the colony acquires a mucous morphology (Lev⁺ phenotype) associated with the formation of the polysaccharide levan. Plasmid pALS200 was incubated with an aqueous solution of hydroxylamine under non-drastic mutagenesis conditions in order to generate a library of LsdA variants with a single amino acid substitution at random positions in the protein sequence. Sequencing of the plasmid recovered from each recombinant *E*. *coli* colony that acquired saccharolytic capacity but failed to produce the mucous morphology (Lev⁻ phenotype), allowed to identify specific changes in 14 amino acids located in the cavity or vicinity of the active site, with a possible direct role in the polymerization reaction. Also in this way, 11 amino acids were identified in positions far from the active site, whose apparent function is to maintain the stability of the enzyme folding. In all cases, the random mutation caused a reduction in enzyme activity, as measured by glucose release, compared to non-mutated LsdA. Active site amino acids R141 and H142 (subsite -1), F274 and N276 (subsite +2), and R304 (subsite +3) were targeted for directed evolution experiments by saturation mutagenesis. These five amino acids are located in regions of the protein that interact with the donor or acceptor substrate.

In the invention, the directed mutagenesis experiments made it possible to establish which amino acid substitution at positions 141, 142, 274, 276 and 304 is optimal, in order to obtain modified variants of LsdA that do not produce the polysaccharide levan and with similar or higher specific activity to the non-mutated enzyme. The most active modified variants within each of the five mutant libraries were purified from *E*. *coli* cell extract and their catalytic properties studied. The analysis of the product profile after incubation with sucrose revealed a differential behavior between the modified variants of LsdA, in terms of FOS yield and composition. The ratio of fructosylation activity versus hydrolysis activity and the ratio of 1-kestotriose versus 6-kestotriose products varied depending on the position and properties of the substitute amino acid.

The saturation mutation of H142 revealed that the preservation or loss of the ability to polymerize FOS to levan varies depending on the type of amino acid incorporated at this position. Modified variants H142A, H142G, and H142N formed high molecular weight levan, just like the non-mutated enzyme. The modified variants H142E and H142P transformed the sucrose substrate via hydrolysis with specific activity slightly higher than non-mutated LsdA. The modified variant H142S, also highly active, kept an acceptable FOS yield but did not completely abolish polysaccharide levan synthesis. The invention also discloses double modified variants of the -1 subsite in which amino acid R141 is substituted to saturation and H142 is replaced to Ser (S). The double substitutions R141K-H142S, R141Y-H142S and R141F-H142S abolished levan formation and caused a lower decrease in 1-kestotriose synthesis comparing to the single mutations H142E and H142P. Sucrose hydrolysis was the main reactions in all levan-deficient variants with mutations at R141 or H1722 (subsite -1).

Amino acids F274 and N276 (+2 subsite) and R304 (+3 subsite) are located in exposed loops towards the surface of the active site cavity, and interact exclusively with the acceptor substrate of the fructosyl residue. The change of F274 by another aromatic amino acid (Y or W) keeps the polymerase activity of LsdA intact. The modified enzymes F274A and F274N synthesized short-chain FOS, and did not polymerize them to levan. Biochemical analysis of the N276 amino acid saturation mutant library revealed that point substitution of Asn (N) at position 276 with a positively charged amino acid such as His (H), Lys (K), or Arg (R) provided modified variants that do not form the polysaccharide levan and differ in spectrum from the FOS products. The N276R substitution stands out for synthesizing 6-kestotriose as the main FOS, unlike non-mutated LsdA, which mainly fructosylates sucrose via the β-(2→1) bond. On the other hand, changing R304 at subsite +3 to Lys (K) resulted in a variant that synthesizes 1-kestotriose and 1,1-kestotetraose as major FOS, in addition to a ladder in decreasing concentration of linear and branched FOS (GF₂₋₁₅) whose chain extends β-(2→6) in a non-processive way. LsdA variants with the double substitutions N276H-R304K, N276K-R304K, and N276R-R304K have high specific activity, synthesize 1-kestotriose and 6-kestotriose in different proportions, and do not form the polysaccharide levan.

In an embodiment of the invention, the modified LsdA enzyme comprising an amino acid sequence selected from the group of sequences composed of SEQ ID NO: 26 - SEQ ID NO: 34 is produced in a genetically modified host microorganism. In a particular embodiment, the genetically modified host microorganism is the yeast *P. pastoris* or the bacterium *E*. *coli.*

Therefore, an object of the invention is a genetically modified microorganism that produces a modified variant that comprises an amino acid sequence selected from the group of sequences composed of SEQ ID NO: 26 - SEQ ID NO: 34. In an embodiment of the invention, it provides recombinant clones of the non-saccharolytic yeast *P. pastoris* that secrete high levels of nine modified variants of LsdA (subsites -1, +2 and +3) that do not produce the polysaccharide levan, selected based on the criteria of higher specific activity and differences in the spectrum of FOS products. The yeast *P. pastoris,* like the bacterium *E*. *coli,* fulfills the requirement of not presenting endogenous enzymes that could react with the sucrose substrate or the FOS products. The occurrence of N-glycosylation in the recombinant enzyme does not cause variation in its catalytic properties, and it does increase its thermal stability. The constitutive expression system used does not require the addition of inducer to the growth medium, so it is suitable for the production of enzymes with application in food processing. This, together with the high initial purity of the recombinant protein in the yeast culture supernatant, makes it possible to obtain an enzyme preparation that meets the technical and regulatory requirements for its commercial application.

The invention, therefore, also provides an enzyme preparation that comprises the modified LsdA enzyme comprising an amino acid sequence selected from the group of sequences composed of SEQ ID NO: 26- SEQ ID NO: 34. The high initial purity of each modified protein in the culture supernatant of the recombinant yeast allowed the obtaining of crude or stable enzymatic preparations of high specific activity. The process for preparing the crude enzyme preparation is feasible to scale up to an industrial level, it includes steps of centrifugation, filtration, concentration, dialysis and lyophilization, well known to those versed in this field of the art. In an embodiment of the invention, the enzyme preparation is characterized by being a liquid or a lyophilisate.

In another aspect, the invention discloses an enzymatic process for conversion of sucrose into FOS that comprises the use of the modified LsdA enzyme comprising an amino acid sequence selected from the group of sequences composed of SEQ ID NO: 26 - SEQ ID NO: 34 or the genetically modified microorganism that produces it.

The invention also reveals a bi-enzymatic process for the conversion of sucrose into FOS where in a first step of the process an enzyme with fructosyltransferase activity is used and in a second step the modified levansucrase is used which comprises an amino acid sequence selected from the group of sequences composed of SEQ ID NO: 26 - SEQ ID NO: 34, the non-mutated levansucrase, or the genetically modified microorganism that produces the modified levansucrase comprising an amino acid sequence selected from the group of sequences composed of SEQ ID NO: 26 - SEQ ID NO: 34. In a particular embodiment, the enzyme used in the first step is of the sucrose:sucrose 1-fructosyltransferase (1-SST) type.

The establishment of a two-step bi-enzymatic cascade reaction process is a novel aspect of the present invention. This process solves the main limitation of the commercial FOS production systems, by allowing the total conversion of the sucrose substrate, producing a mixture of short-chain FOS with different types of linkages, and increasing the yield of total FOS. In a particular embodiment of the invention, the entire process is carried out in a stirred tank with temperature control, this is scalable and easy to implement industrially. By way of illustrating how the process occurs, without constituting a limitation to the invention, in the first step of said process, the recombinant enzyme Sal-SSTrec catalyzes the initial conversion of sucrose at a concentration of 700 g/L into FOS with β-(2→1). At the moment in which the hydrolysis of the 1-kestotriose product begins, the irreversible inactivation of the enzyme is carried out by means of the progressive increase in the temperature of the reactor, from 45°C to 80°C. In the second step of the process, a modified variant of LsdA catalyzes the transformation of the remaining sucrose, broadens the short chain FOS spectrum and increases the total FOS content in the reaction mixture. Added to this improvement in the product profile is an important technological advantage. With the enzymatic removal of sucrose in the reaction mixture, the main contaminant for the chromatographic separation of short-chain FOS is eliminated.

As an example of the process of the invention, a selection of highly active modified variants of LsdA, with different catalytic properties, were used in the conversion of the remaining sucrose substrate when the Sal-SSTrec reaction was stopped. This enzyme synthesizes 1-kestotriose and 1,1-kestotetraose as the main fructosylated products. The magnitude of the increase in FOS yield depends on the ratio of the fructosylation versus hydrolysis activities of the LsdA variant selected for the second reaction. The conversion of the remaining sucrose by the modified variants H142E and R141K-H142S (subsite -1) occurs mainly via hydrolysis, without altering the content and composition of inulin-type FOS produced in the first reaction. The use, in the second reaction, of a modified variant with substitution in positions F274, N276 (subsite +2) and/or R304 (subsite +3) increases the final yield of FOS, while the ratio 1-kestotriose and 1,1-kestotetraose versus β-(2→6)-linked FOS varies depending on the properties of the substitute amino acid.

The invention also reveals the use of the modified levansucarase comprising an amino acid sequence selected from the group of sequences composed of SEQ ID NO: 26 - SEQ ID NO: 34 or the genetically modified microorganism that produces it in an enzymatic process for the conversion of sucrose in FOS.

### Brief description of the figures

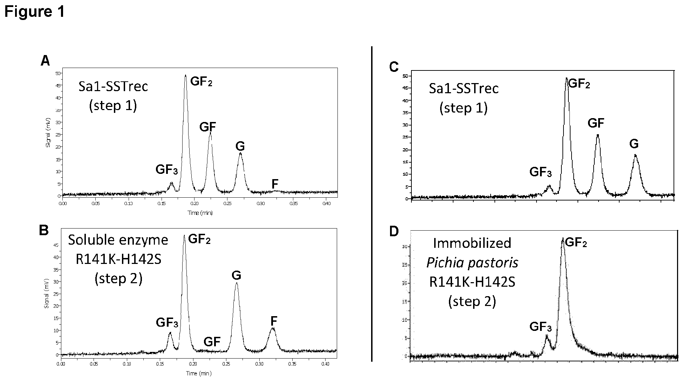

**Figure 1****. High performance liquid chromatography (HPLC) analysis of FOS syrup samples produced in a bi-enzymatic process of two-step reactions.** The chromatograms in panels A and C show the product profile of the intermediate syrup obtained after incubation of the enzyme Sal-SSTrec with a sucrose solution (700 g/L; pH 5.5 and temperature 45°C (step 1). The chromatogram in panel B shows the product profile of the final syrup obtained after the addition of a soluble preparation of modified LsdA with mutations R141K-H142S (step 2) The chromatogram in panel D shows the product profile of the final syrup obtained after the addition of immobilized cells of P. pastoris that express in periplasm the modified LsdA enzyme with mutations R141K-H142S (step 2). Legend: glucose (G), fructose (F), sucrose (GF), kestotriose (GF₂), kestotetraose (GF₃) and kestopentaose (GF₄).

### Examples

### Example 1. Random mutagenesis of the levansucrase gene (lsdA) from Gluconacetobacter diazotrophicus and identification of amino acids involved in the formation of the polysaccharide levan.

*Gluconacetobacter diazotrophicus* levansucrase (designated LsdA) contains a 30 amino acid signal peptide, which is cleaved during transit to the periplasm (Hernández et al., Current Microbiology 39, 146-152, 1999). The transport of the mature enzyme (sequence devoid of the first 30 amino acids) to the external environment is mediated by a type II secretion mechanism and does not involve proteolytic processing (Arrieta et al., J. Bacteriol. 186: 5031-5039, 2014).

Plasmid pALS200 (25 pg), which contains the *lsdA* gene encoding the precursor protein (amino acids 1-584) under the constitutive *nptII* promoter, was incubated with a solution of 0.5 M hydroxylamine-HCl and 5 mM EDTA in 50 mM sodium phosphate buffer (pH 6) for 30 min at 70°C. The mutagenized plasmid was electroporated into *E. coli* strain DH5α. The selection of the transformants was carried out in Petri dishes with solid LB medium (pH 7) supplemented with 0.25% (v/v) glycerol, 5% (p/v) sucrose and the antibiotic ampicillin (100 µg mL⁻¹). After 4-5 days of incubation at 30°C, the phenotype of the transformant colonies was evaluated.

Of a total of approximately 60,000 colonies resistant to the selection antibiotic, 340 were defective in the mucous morphology, associated with the formation of the polysaccharide levan by the action of mature LsdA secreted into the cell periplasm. The 340 non-mucoid colonies were independently inoculated in 24-well plates on solid Luria Broth (LB) medium supplemented with 0.25% (v/v) glycerol, 5% (w/v) sucrose and 0.025% (w/v) bromothymol blue (pH 7), and incubated for 4 days at 30°C. A total of 47 colonies containing mutagenized plasmid and the positive control (*E. coli* with plasmid pALS200 not treated with hydroxylamine) utilized sucrose for growth (Sac⁺ phenotype), evidenced by acidification of the culture medium. In these cases the color of the medium changed from the initial green to yellow. The other recombinant non-mucoid colonies and the negative control (*E. coli* with plasmid without *lsdA* gene) were not saccharolytic. The growth at the expense of yeast extract and tryptone caused the release of ammonium, and the consequent alkalinization of the culture medium. In these cases the color of the culture medium changed from the initial green to blue. The transition range of bromothymol blue pH indicator is yellow (pH<6.0), green (pH 6.0-7.6) and blue (pH>7.6).

In order to differentiate the degree of affectation in the formation of the polysaccharide levan of the 47 colonies that express active modified variants of LsdA, a scale for Lev phenotype was established between 0 (non-mucous colony) and 5 (very mucous colony). The non-mutated LsdA-producing *E*. *coli* colony has the maximal value of 5. Plasmid was recovered from each saccharolytic colony with Lev 0-3, and the region corresponding to the *lsdA* gene was sequenced.

Analysis of deoxyribonucleic acid (DNA) sequencing results revealed 26 independent modified variants possessing a single amino acid substitution in the primary structure of the protein. In one case the substituted amino acid matched but the substitute amino acid (R141C/H) changed. The 25 amino acids involved in the formation of the polysaccharide levan that were identified via random mutagenesis were mapped to the 3D structure of LsdA available in databases (PDB:1W18). Structural modeling positioned 14 amino acids (R141, H142, S198, R199, T213, F274, N276, E284, E297, R304, A339, D368, H389 and G402) in the cavity or vicinity of the active site and 11 amino acids (S179, H255, G263, M294, G315, E324, P359, A394, R412, S413 and P417) in other regions of the protein that are not directly involved in the catalytic process.

In order to evaluate the effect of each mutation on the total activity of the enzyme, the non-mutated LsdA and the 26 modified variants produced in E. coli were purified (Table 1). For this, each recombinant clone of E. coli was grown in a flask with 100 mL of LB medium supplemented with 2% (v/v) glycerol, until stationary phase, in a rotary shaker at 30°C. The biomass was harvested by centrifugation, and resuspended in 50 mM sodium acetate buffer (pH 5.5). Cell disruption was performed by ultrasound, using a Branson-type sonicator (Ultrasonics Corp. Danbury, CT, USA) and the soluble fraction was recovered. The recombinant protein fused to a polyhistidine tag was homogeneously purified by immobilized metal ion affinity chromatography (IMAC) using HisPur^{™} Ni-NTA resin (Thermo Scientific^{™}, USA). The enzyme activity on sucrose was determined by measurements of glucose, a product that is released both in the hydrolysis reaction and in the fructosylation reaction. One unit (U) represents the amount of enzyme that releases 1 pmol of glucose per min, in a reaction at initial rates, with the substrate sucrose at a concentration of 1.5 M in sodium acetate buffer at 100 mM (pH 5.5) and at a temperature of 40°C. Glucose quantification was performed with the GOPOD kit (Megazyme, Ireland).

Protein quantification was performed by the Bradford method (Anal. Biochem. 72: 248-254, 1976), and fraction V of bovine serum albumin was used as standard.

Table 1 shows the main characteristics of the mutated LsdA variants that were defective in the formation of the polysaccharide levan. All modified variants were less active than the non-mutated enzyme. The active site amino acids whose substitution caused total or partial loss of mucous morphology of the recombinant *E. coli* colony (Lev 0-2 phenotype) and/or less detriment of total enzymatic activity were R141, H142, F274, N276 and R304. Amino acids R141 and H142 are located in loop 1 (βIB-βIC), towards the interior of the active site cavity of LsdA, where H142 directly interacts with carbon 6 of the fructosyl residue of the donor substrate (subsite -1) (Martinez-Fleites et al., Biochem. J. 390: 19-27, 2005). Amino acids F274 and N276 (subsite +2) are located in loop 4 (βIID-βIIIA) that lines from the surface to the interior of the active site cavity, where they can interact directly or indirectly with the second monosaccharide (glucose or fructose) of the fructosyl acceptor molecule. Amino acid R304 (subsite +3) is located in loop 5 (IIIB-IIIC) located on the surface of the active site cavity, where it can interact directly or indirectly with the third monosaccharide of the fructosyl acceptor molecule.

Amino acids R141, H142, F274, N276 and R304 were selected as targets for directed evolution experiments by saturation mutagenesis.

**Table 1. Characteristics of randomly mutated LsdA variants.**

| **Random mutation** | **Lev phenotyp e** | **Relative Activity (%)** | **Location in the 3D structure** | **Position in the cavity of the active site** |
|---|---|---|---|---|
| native | 5 | 100 | ---- | |
| R141C | 1 | 45 | loop 1 βIB-βIC | inside (subsite -1) |
| R141H | 1 | 39 | loop 1 βIB-βIC | inside (subsite -1) |
| H142Y | 0 | 35 | loop 1 βIB-βIC | inside (subsite -1) |
| S179N | 2 | 27 | loop 2 βID-βIIA | no* |
| S198L | 3 | 23 | βIIA | interior |
| R199H | 3 | 19 | βIIA | interior |
| T213I | 0 | 9 | βIIB | interior |
| H255Y | 2 | 13 | βIID | no* |
| G263D | 3 | 28 | loop 4 βIID-βIIIA | no* |
| F274A | 1 | 83 | loop 4 βIID-βIIIA | surface (subsite +2) |
| N276H | 2 | 87 | loop 4 βIID-βIIIA | inside (subsite +2) |
| E284K | 3 | 40 | βIIIA | surface |
| M294I | 2 | 9 | βIIIB | no* |
| E297K | 0 | 9 | βIIIB | inside |
| R304C | 2 | 89 | loop 5 IIIB-IIIC | surface (subsite +3) |
| G315D | 2 | 23 | loop 5 βIIIB-IIIC | no* |
| E324K | 2 | 44 | loop 5 βIIIB-IIIC | no* |
| A339V | 0 | 8 | βIIIC | surface |
| P359S | 3 | 15 | βIIID | no* |
| D368V | 2 | 37 | loop 6 βIIID-βIVA | interior |
| H389Y | 0 | 16 | loop 7 βIVB-βIVC | inside (subsite +1) |
| A394V | 3 | 18 | loop 7 βIVB-βIVC | no* |
| G402S | 2 | 8 | βIVC | inside |
| R412H | 3 | 43 | βIVD | no* |
| S413R | 0 | 8 | βIVD | no* |
| P417L | 1 | 13 | βIVD | no* |

| | | | | |
|---|---|---|---|---|
| The asterisk (*) indicates that the amino acid is not located in the active site. | | | | |

### Example 2. Saturation mutagenesis of amino acid H142 in the -1 subsite of LsdA.

The modified variants R141C, R141H and H142Y, obtained by random mutagenesis, do not produce the polysaccharide levan, but showed more than 50% reduction in total activity on sucrose compared to the non-mutated enzyme (see Table 1). Amino acid H142 interacts directly with the Fru-O6' position of the donor sucrose molecule of the fructosyl residue (subsite -1) and is therefore an interesting target for mutagenesis studies.

To assess the effect of each possible amino acid substitution at position 142 on the polysaccharide levan synthesis reaction, a library of LsdA variants was constructed by saturation. Plasmid pALS200 as template and the primers 5' GGATCGTTTCCCATGGCGCATGTAC (SEQ ID NO: 1) and 5' GATGCGGGCATGCACGTG(A/C/G/T)(A/C/G/T)(A/C/G/T)GTCGTCGAAACC (SEQ ID NO: 2) were used in mutagenic PCR. The mutagenic reverse primer contains the CAC triplet degeneracy (H142), and maintains the *SphI* restriction site. The PCR conditions were: 1) 5 min at 95°C, 2) 1 min at 95°C, 3) 1 min at 60°C, 4) 1 min at 72°C, 5) 5 min at 72°C, 30 cycles from step 2 to 4. The amplified product was *NcoI-SphI* digested, and the fragment population (524 bp) was used to replace the corresponding fragment in plasmid pALS200. The ligation mixture was introduced into *E*. *coli* strain DH5α by electroporation. A number of 36 independent transformations were carried out. Transgenic colonies were selected on Petri dishes with solid LB medium (pH 7), supplemented with 0.25% (v/v) glycerol, 5% (w/v) sucrose and the ampicillin antibiotic (100 µg mL⁻¹). After 4-5 days of incubation at 30°C the phenotype of the transformant colonies was evaluated.

Around 1000 ampicillin-resistant colonies, with different degrees of mucus, were plated independently in 24-well plates with solid LB medium supplemented with 5% (w/v) sucrose and 0.025% (w/v) bromothymol blue (pH 7.0), and incubated for 4 days at 30°C. From the colonies with the Sac+ phenotype, the plasmid was purified and the entire region corresponding to the mutated and assembled *lsdA* gene was sequenced. The analysis of the DNA sequencing results confirmed the generation of the 19 possible modified variants with substitution of the amino acid H142. Each LsdA variant was purified by IMAC, from the cell extract of the corresponding *E*. *coli* clone, and the specific activity (U/mg) was quantified based on the initial rate of glucose released (V_{G}) from sucrose at a concentration of 1.5 M in 100 mM sodium acetate buffer (pH 5.5) and a temperature of 40°C. The relationship between the initial rates of kestotriose synthesis (V_{K}) and the release of fructose into water (V_{F}) allowed establishing the ratio of transferase versus hydrolase (T/H) activities.

The profile of reaction products at the end time (24 h) was determined by HPLC, using an Aminex HPX-42 column (Biorad) coupled to a refractive index type detector. Distilled water was used as solvent at a flow rate of 0.5 mL min-1, and the operating temperature was 80°C. Samples were 10-fold diluted and the injection volume was 20 µL. A mixture of glucose (G), fructose (F), sucrose (GF), 1-kestotriose (GF2) and 1,1-kestotetraose (GF3), prepared at the same concentration (40 mg mL⁻¹) of each sugar, was used as standard. The final composition of carbohydrates in the reaction mixture was glucose (G), fructose (F), sucrose (GF), trisaccharide (GF2), tetrasaccharide (GF3), pentasaccharide (GF4), and oligofructans with degrees of polymerization between 6 and 11 (GF₅₋₁₀).

Table 2 shows the main characteristics of the 19 LsdA variants obtained by saturation mutagenesis of amino acid H142. *E*. *coli* colonies expressing the modified variants H142A, H142G and H142N showed the same mucoid morphology (phenotype Lev 5) as the bacteria containing the non-mutagenized plasmid pALS200. A total of 9 modified variants produced non-mucous colonies (Lev 0 phenotype), where H142E and H142P stand out, for presenting specific activity values slightly higher than the non-mutated enzyme, although the transfer of fructose to water is the predominant reaction. The H142S substitution, although it does not completely inhibit the formation of the polysaccharide levan (Lev 3 phenotype), favors the highest yield of total FOS, and the predominance of GF₂ is maintained.

**Table 2. Relevant characteristics of LsdA variants with saturated substitutions of H142.**

| **LsdA variant** | **Lev phenotype (0-5)** | **Specific activity (U/mg)** | **Ratio T / H (V_{K}/V_{F})** | **Product profile at the end of the reaction (%, weight/ weight)** | | | | | | |
|---|---|---|---|---|---|---|---|---|---|---|
| | | | | **G** | **F** | **GF** | **GF₂** | **GF₃** | **GF₄** | **GF₅₋₁₀** |
| native | 5 | 1300 | 0.82 | 39.5 | 20.8 | 2.8 | 16.9 | 5.2 | 0.8 | 10.63 |
| H142Y | 1 | 455 | nd | nd | nd | nd | nd | nd | nd | nd |
| H142L | 1 | 72 | nd | nd | nd | nd | nd | nd | nd | nd |
| H142K | 0 | 325 | nd | nd | nd | nd | nd | nd | nd | nd |
| H142R | 0 | 262 | nd | nd | nd | nd | nd | nd | nd | nd |
| H142A | 5 | 1079 | nd | nd | nd | nd | nd | nd | nd | nd |
| H142G | 5 | 942 | nd | nd | nd | nd | nd | nd | nd | nd |
| H142N | 5 | 897 | nd | nd | nd | nd | nd | nd | nd | nd |
| H142S | 3 | 1289 | 0.64 | 39.2 | 22.5 | 1.8 | 20.2 | 6.7 | 4.2 | 4.85 |
| H142P | 0 | 1325 | 0.16 | 47.4 | 45.8 | 0.9 | 4.2 | 1.1 | 0 | 0 |
| H142E | 0 | 1390 | 0.14 | 48.2 | 46.4 | 0.5 | 3.9 | 0.9 | 0 | 0 |
| H142M | 3 | 301 | nd | nd | nd | nd | nd | nd | nd | nd |
| H142C | 0 | 134 | nd | nd | nd | nd | nd | nd | nd | nd |
| H142D | 0 | 988 | nd | nd | nd | nd | nd | nd | nd | nd |
| H142Q | 3 | 356 | nd | nd | nd | nd | nd | nd | nd | nd |
| H142I | 1 | 455 | nd | nd | nd | nd | nd | nd | nd | nd |
| H142F | 0 | 195 | nd | nd | nd | nd | nd | nd | nd | nd |
| H142T | 1 | 390 | nd | nd | nd | nd | nd | nd | nd | nd |
| H142W | 0 | 186 | nd | nd | nd | nd | nd | nd | nd | nd |
| H142V | 0 | 99 | nd | nd | nd | nd | nd | nd | nd | nd |

| | | | | | | | | | | |
|---|---|---|---|---|---|---|---|---|---|---|
| Legend: G, glucose; F, fructose; GF, sucrose; GF₂, kestotriose; GF₃, kestotetraose; GF₄, kestopentaose; GF₅₋₁₀ FOS with degree of polymerization (DP) between 6 and 11; nd, no determined. Values are means (n=3) and the standard deviation was below 10%. | | | | | | | | | | |

### Example 3. Saturation mutagenesis of amino acid R141 keeping the H142S substitution fixed.

In order to obtain a variant of LsdA mutated in the -1 subsite that does not produce the polysaccharide levan, maintains a high specific activity and increases the yield of FOS, a double mutant library was constructed where the H142S substitution is combined with each of the possible amino acid changes at position 141. The saturation mutagenesis of amino acid R141 was performed by PCR, using the plasmid pALS200 as template and the forward primers 5' GGATCGTTTCCCATGGCGCATGTAC (SEQ ID NO: 1) and reverse 5'GATGCGGGCATGCACGCT(A/C/G/T)(A/C/G/T)(A/C/G/T)GTCGTCGAAACC (SEQ ID NO: 3). The reverse primer maintains the change of the triplet CAC by AGC (H142S), presents degeneration of the triplet CGC (R141) and contains the restriction site *Sph*I*.* PCR conditions are described in Example 2. The population of amplified 593-bp fragments were digested *Nco*I*-Sph*I*,* and inserted into the corresponding region of plasmid pALS200. The ligation mixture was introduced into *E. coli* strain DH5α by electroporation. A number of 36 independent transformations were carried out. Transgenic colonies were selected on Petri dishes with solid LB medium (pH 7), supplemented with 0.25% (v/v) glycerol, 5% (w/v) sucrose and the ampicillin antibiotic (100 µg mL⁻¹). After 4-5 days of incubation at 30°C the phenotype of the transformant colonies was evaluated.

Over 1000 ampicillin-resistant colonies were plated independently on 24-well plates with solid LB medium supplemented with 5% (w/v) sucrose and 0.025% (w/v) bromothymol blue (pH 7), and incubated for 4 days at 30°C. All transgenic colonies were saccharolytic (Sac⁺ phenotype) and the degree of mucoid morphology (Lev phenotype) ranged between 0 and 3. Sequencing of the region corresponding to the *lsdA* gene in the plasmids recovered from independent transformants revealed the generation of all 19 amino acid substitution variants R141 in combination with the fixed mutation H142S. Each modified LsdA variant was purified by IMAC from the cell extract of the corresponding *E. coli* clone The specific activity (U/mg), the ratio of transferase versus hydrolase activities (T/H), and the composition of the reaction products were determined as described in Examples 1 and 2. Table 3 shows the main characteristics of the LsdA variants that contain each of the 19 possible substitutions of the amino acid R141 and keep the H142S substitution fixed. E. *coli* colonies expressing LsdA variants with double substitutions where amino acid R141 changed to N, G, C, Q, H, E, D, T, or M retained some degree of mucoid morphology (Lev 2-3 phenotype). The rest of the double variants produced non-mucoid colonies (Lev 0 phenotype), where the combinations K141-S142, Y141-S142, F141-S142, V141-S142, L141-S142 and A141-S142 were distinguished by presenting similar specific activity values to the non-mutated enzyme. These six modified variants that do not produce the polysaccharide levan fructosylated sucrose to kestotriose (GF2) and to a lesser extent kestotetraose (GF3), although the transfer of fructose to water continued to be the predominant reaction. Among the LsdA variants unable to polymerize FOS to levan, the double substitution K141-S 142 caused the least reduced value in the ratio of transfructosylation versus hydrolysis.

**Table 3. Relevant characteristics of LsdA variants with saturated mutation of H142 and the substitution H172S.**

| **LsdA variant** | **Lev phenotype (0-5)** | **Specific activity (U/mg)** | **Ratio T / H (V_{K}/V_{F})** | **Product profile at the end of the reaction (%, weight/ weight)** | | | | | | |
|---|---|---|---|---|---|---|---|---|---|---|
| | | | | **G** | **F** | **GF** | **GF₂** | **GF₃** | **GF₄** | **GF₅₋₁₀** |
| R141-S142 | 3 | 1289 | 0.64 | 39.2 | 22.5 | 1.8 | 20.2 | 6.7 | 4.2 | 4.85 |
| V141-S142 | 0 | 1270 | 0.27 | 45.9 | 42.7 | 2.2 | 7.6 | 0.9 | 0 | 0 |
| K141-S142 | 0 | 1347 | 0.38 | 44.6 | 40.5 | 1.9 | 8.9 | 3.4 | 0 | 0 |
| L141-S142 | 0 | 1240 | 0.21 | 45.8 | 42.4 | 3.3 | 7.1 | 0.6 | 0 | 0 |
| Y141-S142 | 0 | 1265 | 0.32 | 45.1 | 41.1 | 2.9 | 8.2 | 2.7 | 0 | 0 |
| P141-S142 | 0 | 266 | 0.12 | nd | nd | nd | nd | nd | nd | nd |
| N141-S142 | 3 | 701 | 0.66 | nd | nd | nd | nd | nd | nd | nd |
| S141-S142 | 0 | 623 | 0.26 | nd | nd | nd | nd | nd | nd | nd |
| A141-S142 | 0 | 1293 | 0.18 | 46.3 | 43.2 | 2.8 | 5.5 | 1.2 | 0 | 0 |
| G141-S142 | 3 | 595 | 0.54 | nd | nd | nd | nd | nd | nd | nd |
| F141-S142 | 0 | 1260 | 0.35 | 44.9 | 40.6 | 3.2 | 8.6 | 2.5 | 0 | 0 |
| I141-5142 | 0 | 514 | 0.21 | nd | nd | nd | nd | nd | nd | nd |
| W141-S142 | 0 | 127 | 0.33 | nd | nd | nd | nd | nd | nd | nd |
| C141-S142 | 3 | 645 | 0.47 | nd | nd | nd | nd | nd | nd | nd |
| Q141-S142 | 3 | 795 | 0.61 | nd | nd | nd | nd | nd | nd | nd |
| H141-S142 | 3 | 673 | 0.72 | nd | nd | nd | nd | nd | nd | nd |
| E141-S142 | 3 | 534 | 0.48 | nd | nd | nd | nd | nd | nd | nd |
| D141-S142 | 2 | 351 | 0.31 | nd | nd | nd | nd | nd | nd | nd |
| T141-S142 | 3 | 843 | 0.42 | nd | nd | nd | nd | nd | nd | nd |
| M141-S142 | 2 | 480 | 0.33 | nd | nd | nd | nd | nd | nd | nd |

| | | | | | | | | | | |
|---|---|---|---|---|---|---|---|---|---|---|
| The specific activity (U/mg), the ratio of transferase versus hydrolase activities (T/H), and the composition of the reaction products were determined as described in Examples 1 and 2. Legend: G, glucose; F, fructose; GF, sucrose; GF₂, kestotriose; GF₃, kestotetraose; GF₄, kestopentaose; GF₅₋₁₀ FOS with degree of polymerization (DP) between 6 and 11; nd, no determined. Values are means (n=3) and the standard deviation was below 10%. | | | | | | | | | | |

### Example 4. Site-directed mutagenesis of amino acid F274 in the +2 subsite of LsdA.

*E. coli* colonies expressing the modified variant F274A generated by random mutagenesis use sucrose as a carbon source (Sac+ phenotype) and present a notable affectation of the mucoid morphology associated with the production of the polysaccharide levan (phenotype Lev 1) (see Table 1). The active site residue F274 occupies a position that must interact with the second monosaccharide of the fructosyl acceptor (subsite +2) and it is therefore an interesting target for site-directed mutagenesis studies.

The modified variant F274A is 17% less active than the non-mutated enzyme (see Table 1). In order to optimize the mutation at position 274, the original amino acid F was replaced by N, Y and W using the *overlapping PCR* technique. Plasmid pAL200 was used as template, and the following oligonucleotides as primers, where the underlined triplet corresponds to the designed mutation.

| |
|---|
| *Bam*HI-foward 5'CTGGGGC***GGATCC***ACGCCGACTTC (SEQ ID NO: 4) |
| F274N-foward 5'GCGCAGAACGAAAACTTCAATTTCCGCGATCCG (SEQ ID NO: 5) |
| F274N-reverse 5'CGGATCGCGGAAATTGAAGAATTCGTTCTGCGC (SEQ ID NO: 6) |
| F274Y-foward 5'GCGCAGAACGAATACTTCAATTTCCGCGATCCG (SEQ ID NO: 7) |
| F274Y- reverse 5'CGGATCGCGGAAATTGAAGTATTCGTTCTGCGC (SEQ ID NO: 8) |
| F274W-foward 5'GCGCAGAACGAATGGTTCAATTTCCGCGATCCG (SEQ ID NO: 9) |
| F274W- reverse 5'CGGATCGCGGAAATTGAACCATTCGTTCTGCGC (SEQ ID NO: 10) |
| *Kpn*I-reverse 5'GGCGCCAG***GGTACC***CCCGCGACGG (SEQ ID NO: 11) |

The PCR conditions were: 1) 95°C 5 min, 2) 95°C 1 min, 3) 60°C 1 min, 4) 72°C 1 min, 5) 72°C 5 min, 30 cycles from step 2 to 4. The amplified PCR product containing each designed mutation was digested with *BamH*I*-Kpn*I*,* and the resulting fragment (782 bp) was used to replace the corresponding fragment in plasmid pALS200. The three new constructs were independently introduced into *E. coli* strain DH5α. All ampicillin-resistant transformants acquired the ability to use sucrose as a carbon source (Sac+ phenotype). The mucoid degree of the sucrose-utilizing colonies varied depending on the type of mutation. The replacement of F274 by N, Y and W was verified by sequencing the plasmid recovered from the corresponding transformant. The variants F274N, F274Y, F274W and F274A containing a polyhistidine tag were purified to homogeneity from *E. coli* extracts by immobilized metal ion affinity chromatography (IMAC). The specific activity (U/mg), the ratio of transferase versus hydrolase activities (T/H), and the composition of the reaction products were determined as described in Examples 1 and 2.

Table 4 shows the catalytic behavior of the four generated LsdA variants with punctual substitution of the amino acid F274. The product profile of the reaction with sucrose at initial concentration of 1.5 M was determined by HPLC, as described in Example 2. The substitution of F274 for another aromatic amino acid (Y or W) did not affect the production of the polysaccharide levan, nor did it increase the yield of total FOS with respect to the non-mutated enzyme. The reaction of F274A and F274N with sucrose at a concentration of 1.5 M yielded short-chain FOS but not the polysaccharide levan.

**Table 4. Relevant characteristics of four LsdA variants with replacement of amino acid F274**

| **LsdA variant** | **Lev phenotype (0-5)** | **Specific activity (U/mg)** | **Ratio T/H (V_{K}/V_{F})** | **Product profile at the end of the reaction (%, weight/ weight)** | | | | | | |
|---|---|---|---|---|---|---|---|---|---|---|
| | | | | **G** | **F** | **GF** | **GF₂** | **GF₃** | **GF₄** | **GF₅₋₁₀** |
| Non-mutated | 5 | 1300 | 0.82 | 39.5 | 20.8 | 2.8 | 16.9 | 5.2 | 0.8 | 10.63 |
| F274N | 1 | 1020 | 0.59 | 40.6 | 31.5 | 3.1 | 17.1 | 5.4 | 1.1 | 0.3 |
| F274Y | 5 | 789 | 0.88 | nd | nd | nd | nd | nd | nd | nd |
| F274W | 5 | 556 | 0.77 | nd | nd | nd | nd | nd | nd | nd |
| F274A | 1 | 1079 | 0.64 | 39.8 | 29.1 | 3.3 | 19.2 | 6.1 | 1.2 | 0.8 |

| | | | | | | | | | | |
|---|---|---|---|---|---|---|---|---|---|---|
| The specific activity (U/mg), the ratio of transferase versus hydrolase activities (T/H), and the composition of the reaction products were determined as described in Examples 1 and 2. Legend: G, glucose; F, fructose; GF, sucrose; GF₂, kestotriose; GF₃, kestotetraose; GF₄, kestopentaose; GF₅₋₁₀ FOS with degree of polymerization (DP) between 6 and 11; nd, no determined. Values are means (n=3) and the standard deviation was below 10%. | | | | | | | | | | |

**Example 5. Saturation mutagenesis of amino acid N276 in the +2 subsite of LsdA.** *E. coli* colonies expressing the modified variant N276H of LsdA, obtained by random mutagenesis, use sucrose as a carbon source (Sac+ phenotype) and present a notable affectation of the mucosal appearance associated with the production of the polysaccharide levan (phenotype Lev 2) (see Table 1). The active site residue N276 occupies a position of possible interaction with the second monosaccharide of the acceptor molecule of the fructosyl residue (subsite +2) and is therefore an interesting target for site-directed mutagenesis studies. The modified variant N276H is 13% less active than the non-mutated enzyme (see Table 1). To assess the effect of each of the possible 19 amino acid substitutions at position 276 on the formation of polysaccharide levan, a library of LsdA variants was constructed by saturation mutagenesis using PCR. The reaction was performed using plasmid pALS200 as template, the forward primer 5'CGCAGAACGAATTCTTC(A/C/G/T)(A/C/G/T)(A/C/G/T)TTCCGCG (SEQ ID NO: 12) (maintains the *EcoR*I restriction site and contains the degeneracy of the AAT triplet encoding N276) and the reverse primer 5'GGCGCCAGGGTACCCCCGCGACGG (SEQ ID NO: 11) (maintains the *Kpn*I restriction site). The mutagenic PCR conditions were: 1) 95°C 5 min, 2) 95°C 1 min, 3) 60°C 1 min, 4) 72°C 1 min, 5) 72°C 5 min, 30 cycles from step 2 to 4.

The amplified product was digested by *EcoR*I*-Kpn*I*,* and the resulting 616-bp fragment was used to replace the corresponding fragment in plasmid pALS200. The ligation mix was electroporated into *E. coli* strain DH5α. Twenty independent transformations were performed, and the selection of the transforming colonies was carried out in Petri dishes with solid LB medium (pH 7.0) supplemented with 0.25% (v/v) glycerol, 5% (w/v) sucrose and the antibiotic ampicillin (100 µg mL⁻¹). After 4-5 days of incubation at 30°C the phenotype of the transformants was evaluated.

A total of approximately 1000 ampicillin-resistant colonies, with different degrees of mucoid morphology, were plated independently in 24-well plates with solid LB medium supplemented with 5% (w/v) sucrose and 0.025% (w/v) bromothymol blue (pH 7.0) and incubated for 4 days at 30°C. Plasmid was recovered from colonies with Sac⁺ phenotype and the region corresponding to the *lsdA* gene was sequenced. DNA sequencing confirmed the generation of all 19 substitution variants of amino acid N276. Each LsdA variant was purified by IMAC from the cell extract of the corresponding *E. coli* clone. The specific activity (U/mg), the ratio of transferase versus hydrolase activities (T/H), and the composition of the reaction products were determined as described in Examples 1 and 2.

Table 5 shows the main characteristics of the modified LsdA variants with the saturated substitution of the amino acid N276. The product profile of the reaction with sucrose at initial concentration of 1.5 M was determined by HPLC, as described in Example 2. *E. coli* transformants showed degrees of mucoid morphology (Lev phenotype) ranging from 0 to 5. The variants containing the substitution of Asn (N) for Pro (P), Asp (D), Gly (G) or Ala (A) at position 276 showed the Lev 5 phenotype, indicating that levan formation was not affected. The modified variant N276H was slightly more active than the non-mutated enzyme, and produced colonies with a lower degree of mucoid morphology (phenotype Lev 2). Among the modified variants that produced non-mucoid colonies (Lev 0 phenotype), the N276R and N276K variants stand out, with a specific activity similar to that of the non-mutated enzyme and a higher yield of total FOS.

**Table 5. Relevant characteristics of LsdA variants with saturated substitution of amino acid F274**

| **LsdA variant** | **Lev phenotype (0-5)** | **Specific activity (U/mg)** | **Ratio T / H (*V*_{K}/*V*_{F})** | **Product profile at the end of the reaction (%, weight/ weight)** | | | | | | |
|---|---|---|---|---|---|---|---|---|---|---|
| | | | | **G** | **F** | **GF** | **GF₂** | **GF₃** | **GF₄** | **GF₅₋₁₀** |
| Non-mutated | 5 | 1300 | 0.82 | 39.5 | 20.8 | 2.8 | 16.9 | 5.2 | 0.8 | 10.63 |
| N276R | 0 | 1269 | 0.64 | 39.8 | 30.1 | 3.2 | 18.3 | 7.9 | 0.3 | 0 |
| N276K | 0 | 1260 | 0.63 | 40.4 | 30.9 | 3.2 | 17.7 | 7 | 0.2 | 0 |
| N276Y | 0 | 130 | 0.54 | nd | nd | nd | nd | nd | nd | nd |
| N276V | 3 | 1025 | 0.63 | nd | nd | nd | nd | nd | nd | nd |
| N276M | 2 | 1257 | 0.53 | 42.6 | 34.7 | 3.9 | 8.4 | 6.9 | 1.7 | 0.6 |
| N276H | 2 | 1397 | 0.72 | 40.1 | 28.2 | 1.6 | 19.4 | 9.3 | 1 | 0.2 |
| N276A | 5 | 1253 | 0.83 | nd | nd | nd | nd | nd | nd | nd |
| N276T | 3 | 1019 | 0.61 | nd | nd | nd | nd | nd | nd | nd |
| N276C | 4 | 1023 | 0.73 | nd | nd | nd | nd | nd | nd | nd |
| N276S | 0 | 8 | nd | nd | nd | nd | nd | nd | nd | nd |
| N276G | 5 | 771 | 0.77 | nd | nd | nd | nd | nd | nd | nd |
| N276E | 3 | 1006 | 0.71 | nd | nd | nd | nd | nd | nd | nd |
| N276Q | 4 | 838 | 0.66 | nd | nd | nd | nd | nd | nd | nd |
| N276D | 5 | 816 | 0.79 | nd | nd | nd | nd | nd | nd | nd |
| N276I | 1 | 720 | 0.47 | nd | nd | nd | nd | nd | nd | nd |
| N276L | 0 | 132 | nd | nd | nd | nd | nd | nd | nd | nd |
| N276F | 0 | 11 | nd | nd | nd | nd | nd | nd | nd | nd |
| N276P | 5 | 535 | 0.93 | nd | nd | nd | nd | nd | nd | nd |
| N276W | 0 | 68 | nd | nd | nd | nd | nd | nd | nd | nd |

The specific activity (U/mg), the ratio of transferase versus hydrolase activities (T/H), and the composition of the reaction products were determined as described in Examples 1 and 2. Legend: G, glucose; F, fructose; GF, sucrose; GF₂, kestotriose; GF₃, kestotetraose; GF₄, kestopentaose; GF₅₋₁₀ FOS with degree of polymerization (DP) between 6 and 11; nd, no determined. Values are means (n=3) and the standard deviation was below 10%.

### Example 6. Site-directed mutagénesis of amino acid R304 in subsite +3

*E. coli* colonies expressing the modified LsdA variant R304C, obtained by random mutagenesis, use sucrose as a carbon source (Sac+ phenotype) and present a notable affectation of the mucoid morphology (phenotype Lev 2) associated with the production of the polysaccharide levan (see Table 1). The position of amino acid R304 at the surface of the active site cavity suggests possible interaction with the third monosaccharide of the acceptor molecule (subsite +3). Thus, R334 is an interesting target for site-directed mutagenesis studies.

The modified variant R304C is 11% less active than the non-mutated enzyme (see Table 1). In order to optimize the mutation at position 304, the original amino acid R was replaced by H, K, S and A. The four modified variants were constructed using the overlapping PCR technique, the pALS200 plasmid as template, and the following oligonucleotides as primers, where the underlined triplet corresponds to the designed mutation.

| | |
|---|---|
| *BamHI*-forward | 5'CTGGGGC***GGATCC***ACGCCGACTTC (SEQ ID NO: 4) |
| R304H-forward | 5'GGCAATACCGCGGGCCAGCATGGCGTCGCCAAC (SEQ ID NO: 13) |
| R304H-reverse | 5'GTTGGCGACGCCATGCTGGCCCGCGGTATTGCC (SEQ ID NO: 14) |
| R304K-forward | 5'GGCAATACCGCGGGCCAGAAGGGCGTCGCCAAC (SEQ ID NO: 15) |
| R304K-reverse | 5'GTTGGCGACGCCCTTCTGGCCCGCGGTATTGCC (SEQ ID NO: 16) |
| R304S-forward | 5'GGCAATACCGCGGGCCAGTCGGGCGTCGCCAAC (SEQ ID NO: 17) |
| R304S-reverse | 5'GTTGGCGACGCCCGACTGGCCCGCGGTATTGCC (SEQ ID NO: 18) |
| R304A-forward | 5'GGCAATACCGCGGGCCAGGCCGGCGTCGCCAAC (SEQ ID NO: 19) |
| R304A-reverse | 5'GTTGGCGACGCCGGCCTGGCCCGCGGTATTGCC (SEQ ID NO: 20) |
| *Kpn*I-reverse | 5'GGCGCCAG***GGTACC***CCCGCGACGG (SEQ ID NO: 11) |

The PCR conditions were: 1) 95°C 5 min, 2) 95°C 1 min, 3) 60°C 1 min, 4) 72°C 1 min, 5) 72°C 5 min, 30 cycles from step 2 to 4. Each PCR product containing the designed mutation was *BamH*I*-Kpn*I digested and the resulting 717-bp fragment was used to replace the corresponding fragment in plasmid pALS200. The four constructs were independently introduced into *E. coli* strain DH5α. In all cases, the transformants acquired the ability to utilize sucrose. Depending on the type of substitution, the mucoid morphology of the colonies (Lev phenotype) varied from degree 2-4. DNA sequencing confirmed the point substitution of R304 for H, K, S or A.

Modified variants R304H, R304K, R304S and R304A containing a C-terminal polyhistidine tag were purified to homogeneity by IMAC from *E. coli* extracts. The specific activity (U/mg), the ratio of transferase versus hydrolase activities (T/H), and the composition of the reaction products were determined as described in Examples 1 and 2.

Table 6 shows the catalytic behavior of the five LsdA variants with punctual substitution of R304. The product profile of the reaction with sucrose at initial concentration of 1.5 M was determined by HPLC, as described in Example 2. The modified variant R304K showed similar specific activity and higher total FOS yield than the non-mutated enzyme in the reaction with sucrose (1.5 M). The substitution of Arg (R) for the basic Lys (K) caused the smallest decrease in the fructosylation *versus* hydrolysis (F/H) ratio compared to the modified variants R304C, R304H, R304S and R304A. These five variants synthesized GF2, GF3 and a linear FOS ladder (GF4-10) in decreasing concentration that did not lead to the processive synthesis of high molecular weight levan.

**Table 6. Relevant characteristics of five LsdA variants with replacement of amino acid R304**

| **LsdA variant** | **Lev phenotyp e (0-5)** | **Specific activity (U/mg)** | **Ratio T / H (*V*_{K}/*V*_{F})** | **Product profile at the end of the reaction (%, weight/ weight)** | | | | | | |
|---|---|---|---|---|---|---|---|---|---|---|
| | | | | **G** | **F** | **GF** | **GF₂** | **GF₃** | **GF₄** | **GF₅₋₁₀** |
| Non-mutated | 5 | 1300 | 0,82 | 39.5 | 20.8 | 2.8 | 16.9 | 5.2 | 0.8 | 10.63 |
| R304C | 2 | 1157 | 0.66 | 39.7 | 28.1 | 4.2 | 20.2 | 2.4 | 1.6 | 3.2 |
| R304H | 4 | 1280 | 0.74 | 38.2 | 21.3 | 3 | 22.1 | 7.4 | 3.1 | 4 |
| R304K | 4 | 1300 | 0.76 | 38.1 | 23.5 | 2.8 | 22.7 | 7.8 | 1.7 | 3.2 |
| R304S | 4 | 1291 | 0.63 | 39.8 | 25.9 | 2.9 | 19.1 | 5.8 | 2.2 | 3.7 |
| R304A | 4 | 1198 | 0.71 | 38.7 | 24.6 | 3.7 | 19.3 | 6.1 | 4,1 | 3 |

| | | | | | | | | | | |
|---|---|---|---|---|---|---|---|---|---|---|
| The specific activity (U/mg), the ratio of transferase versus hydrolase activities (T/H), and the composition of the reaction products were determined as described in Examples 1 and 2. Legend: G, glucose; F, fructose; GF, sucrose; GF₂, kestotriose; GF₃, kestotetraose; GF₄, kestopentaose; GF₅₋₁₀ FOS with degree of polymerization (DP) between 6 and 11; nd, no determined. Values are means (n=3) and the standard deviation was below 10%. | | | | | | | | | | |

**Example** 7. Construction of modified variants with substitutions at the +2 and +3 subsites. Amino acids N276 (+2 subsite) and R304 (+3 subsite) are located in exposed loops towards the surface of the active site, where direct or indirect interactions with the fructosyl acceptor are established. Based on the results of the specific activity and FOS yield shown in examples 5 and 6, it was decided to construct the double modified variants N276H-R304K, N276R-R304K and N276K-R304K. Site-directed mutagenesis was performed using the PCR technique. Plasmid pALS200 (LsdA-R304K) was used as template and the following oligonucleotides were used as primers. The forward primers contain the EcoRI site (bold italics) and the designed mutation (underlined triplet). The reverse primer contains the *Kpn*I site (bold italics).

| | |
|---|---|
| N276H-forward | 5'CGCAGAAC***GAATTC***TTCCACTTCCGCG (SEQ ID NO: 21) |
| N276R-forward | 5'CGCAGAAC***GAATTC***TTCCGCTTCCGCG (SEQ ID NO: 22) |
| N276K-forward | 5'CGCAGAAC***GAATTC***TTCAAGTTCCGCG (SEQ ID NO: 23) |
| *Kpn*I-reverse | 5'GGCGCCAG***GGTACC***CCCGCGACGG (SEQ ID NO: 11) |

The PCR conditions were: 1) 95°C 5 min, 2) 95°C 1 min, 3) 60°C 1 min, 4) 72°C 1 min, 5) 72°C 5 min, 30 cycles from step 2 to 4. The amplified product containing each mutation was *EcoR*I*-Kpn*I digested and the resulting 616-bp fragment was used to replace the corresponding fragment in plasmid pALS200. Each construct was introduced into *E. coli* strain DH5α. In all three cases, the ampicillin-resistant transformants acquired the ability to use sucrose as a carbon source (Sac+ phenotype) while the colonies showed a non-mucoid morphology (Lev0 phenotype). DNA sequencing confirmed the generation of the double variants N276H-R304K, N276R-R304K without the occurrence of non-designed mutations.

The three double mutants N276H-R304K, N276R-R304K containing a C-terminal polyhistidine tag were purified to homogeneity by IMAC *from E. coli* extracts. The combined effect of substituting N276 (subsite +2) for a basic amino acid (H, R or K) and R304 (subsite +3) for another basic amino acid, but with a lower molecular mass (K), unaltered the specific activity and increased the yield of short-chain FOS. The processive mechanism of FOS polymerization was abolished impeding the formation of high DP levan (Table 7).

**Table 7. Relevant characteristics of the double LsdA mutants with substitutions at N276 and R304.**

| **LsdA variant** | **Lev phenotype (0-5)** | **Specific activity (U/mg)** | **Ratio T / H (V_{K}/V_{F})** | **Product profile at the end of the reaction (%, weight/ weight)** | | | | | | |
|---|---|---|---|---|---|---|---|---|---|---|
| | | | | **G** | **F** | **GF** | **GF₂** | **GF₃** | **GF₄** | **GF₅₋₁₀** |
| Non-mutated | 5 | 1300 | 0.82 | 39.5 | 20.8 | 2.8 | 16.9 | 5.2 | 0.8 | 10.63 |
| N276H-R304K | 0 | 1301 | 0.87 | 39.7 | 29.3 | 1.9 | 19.2 | 8.1 | 1.1 | 0 |
| N276R-R304K | 0 | 1298 | 0.92 | 38.8 | 25.7 | 2.7 | 20.7 | 9.8 | 1.9 | 0 |
| N276K-R304K | 0 | 1296 | 0,85 | 39.2 | 26.9 | 2.8 | 20.3 | 9.6 | 0.7 | 0 |

| | | | | | | | | | | |
|---|---|---|---|---|---|---|---|---|---|---|
| The specific activity (U/mg), the ratio of transferase versus hydrolase activities (T/H), and the composition of the reaction products were determined as described in Examples 1 and 2. Legend: G, glucose; F, fructose; GF, sucrose; GF2, kestotriose; GF3, kestotetraose; GF4, kestopentaose; GF5-10 FOS with degree of polymerization (DP) between 6 and 11; nd, no determined. Values are means (n=3) and the standard deviation was below 10%. | | | | | | | | | | |

### Example 8. Identification of the types of FOS synthesized by a set of modified LsdA variants that do not produce the polysaccharide levan.

The detailed composition of short-chain FOS (DP3-4) synthesized from sucrose (1.5 M) by nine levan-defective LsdA variants was determined using the HPAEC-PAD technique. These modified variants are named H142E (SEQ ID NO: 26), R141K-H142S (SEQ ID NO: 27), F274A (SEQ ID NO: 28), N276H (SEQ ID NO: 29), N276R (SEQ ID NO: 30), R304K (SEQ ID NO: 31), N276H-R304K (SEQ ID NO: 32), N276R-R304K (SEQ ID NO: 33) y N276K-R304K (SEQ ID NO: 34). Samples taken at different reaction times were diluted 50-fold in distilled water, and run on a PA100 Carbopack column (3 x 250 mm) maintained at 30°C. Carbohydrate elution was performed with a 0-60% gradient of 500 mM sodium acetate in 100 mm NaOH, at a flow rate of 0.25 mL min⁻¹. Detection was performed with a Dionex ED40 module using a gold working electrode and a Ag/AgCl reference electrode.

HPAEC-PAD analysis of the product profile in a prolonged reaction revealed that the non-mutated enzyme and the nine modified variants synthesized varied mixtures of FOS with β-(2→1) and β-(2→6) fructosyl linkages (Table 8). Fructosylation of sucrose by the single mutant N276R and the double mutants N276R-R304K and N276K-R304K resulted in a majority formation of 6-kestotriose. In contrast, the rest of the modified variants and the non-mutated enzyme produced 1-kestotriose and 1,1-kestotetraose as the main trisaccharide and tetrasaccharide products, respectively. The contrasting behavior of the modified variants N276R and N276H, regarding the predominant type of linkage in the FOS products, indicates that the +2 subsite plays an important role in determining the orientation of the acceptor molecule, a key factor influencing the regio-selectivity of the fructosyl transfer reaction.

**Table 8. Types of short-chain FOS synthesized by representative variants.**

| **LsdA variant** | **FOS products (% weight/weight)** | | | | | |
|---|---|---|---|---|---|---|
| | **Kestotrioses** | | | **Kestotetraoses** | | |
| | **1K** | **6K** | **NK** | **1,1K** | **1,6K** | **6,6K** |
| Non-mutated | 12.7 | 3 | 1.2 | 4.1 | 0.9 | 0.2 |
| H142E (subsite -1) | 3.7 | 0.1 | 0.1 | 0.9 | 0 | 0 |
| R141K-H142S (subsite -1) | 7.8 | 0.9 | 0.2 | 2.8 | 0.4 | 0.2 |
| F274A (subsite +2) | 10.8 | 6.1 | 2.3 | 3.2 | 0.3 | 2.6 |
| N276H (subsite +2) | 10 | 6.2 | 3.2 | 4.5 | 2.2 | 2.6 |
| N276R (subsite +2) | 6.9 | 10.5 | 0.9 | 0.7 | 2.2 | 5 |
| R304K (subsite +3) | 17.2 | 1.8 | 3.7 | 4.2 | 2.2 | 1.4 |
| N276H-R304K (subsites +2 & +3) | 8.8 | 5.1 | 5.3 | 3.4 | 2.2 | 2.5 |
| N276R-R304K (subsites +2 & +3) | 5.2 | 9.6 | 5.9 | 0.2 | 2.9 | 6.7 |
| N276K-R304K (subsites +2 & +3) | 5.3 | 9.7 | 5.3 | 0.7 | 2.8 | 6.1 |

| | | | | | | |
|---|---|---|---|---|---|---|
| Legend: G, glucose; F, fructose; GF, sucrose; GF2, kestotriose; GF3, kestotetraose; GF4, kestopentaose; GF5-10 FOS with degree of polymerization (DP) between 6 and 11; nd, no determined. Values are means (n=3) and the standard deviation was below 10%. | | | | | | |

### Example 9. Recombinant production of modified LsdA variants in Pichia pastoris.

The non-saccharolytic yeast *P. pastoris* was the eukaryotic host used to evaluate the production of N-glycosylated forms of the nine modified variants of LsdA described in Example 8. In all cases, the 1.7-kb DNA fragment encoding the LsdA mature region (the first 30 amino acids constituting the signal peptide present in the precursor protein are removed) was amplified by PCR. The plasmids harboring the *lsdA* gene with the nine triplet changes under study (Table 9) were the templates in independent reactions using the forward primer 5'AGTGCGCT***ATCGAT***AGGGAATTTCAGC (SEQ ID NO: 24) and the reverse primer 5'TTACTGG***TCTAGA***AATTGGCGAACCTG (SEQ ID NO: 25). PCR conditions were described in Example 2. Each amplified product (gene without the stop codon) was *Cla*I*-Xba*I digested and inserted into the corresponding sites of the pGAPZaC vector (Invitrogen, San Diego, CA, USA). In this way, the mature region of each modified LsdA variant was fused at the N-terminus to the *Saccharomyces cerevisiae* alpha-factor signal peptide and at the C-terminus to the myc epitope and the polyhistidine tag. The hybrid gene is transcribed under the control of the constitutive *P. pastoris* glyceraldehyde 3-phosphate dehydrogenase (GAP) promoter. Each ligation mix was electroporated into *E. coli* strain DH5α and transformants were selected in Luria Broth medium without sodium chloride and supplemented with the antibiotic zeocin (25 µg mL⁻¹). The constructed plasmids were linearized at the *Aur*II site in the *GAP* promoter and introduced by electroporation into *P. pastoris* strain X-33, according to the instructions in the Invitrogen manual (Invitrogen San Diego, CA, USA). Transformants were selected on YPG medium (1% (w/v) yeast extract, 2% (w/v) peptone, 2% (w/v) glycerol, 1.5% (w/v) agar) supplemented with zeocin (100 µg mL-¹). Subsequently, 20 independent colonies of each clone were grown in SYP solid medium [1% (w/v) yeast extract, 2% (w/v) peptone, 5% (w/v) sucrose, 0.025% (w/v) bromthymol blue, pH 6.5)] in order to evaluate the saccharolytic (Sac) and mucoid (Lev) phenotypes. The zeocin-resistant colonies expressing either a modified LsdA variant or the non-mutated enzyme acquired saccharolytic capacity (Sac⁺ phenotype) as evidenced by the change of the medium color from initial green (pH 6.5) to yellow (pH <5.5). The yeast electroporated with the empty vector pGAPZaC vector remained unable to utilize sucrose (Sac- phenotype) and changed the medium color to blue (pH >7.5). All *P. pastoris* transformants that acidified the SYP plates (Suc+ phenotype) secreted an active LsdA variant in shaking batch cultures. The clones with the highest sucrase activity in the cell periplasm and the culture supernatant were chosen to evaluate their performance in fed-batch fermentations.

A 7.5-L fermenter (Marubishi, Japan) containing 3.5 L of medium was inoculated with 200 mL of shaking-batch cultures at logarithmic phase. The same medium was used for the growth of recombinant yeast in a shaker and a fermenter, consisting of: 5% (v/v) glycerol; 0.5% (w/v) yeast extract; 2.2% (w/v) (NH₄)₂SO₄; 1.82% (w/v) K₂HPO₄; 0.75% (w/v) MgSO₄ ·7H₂O; 0.05% (w/v) CaCl₂·2H₂O; vitamins and trace minerals. The fermenter was operated at temperature of 28 °C and pH 5.5. The pH value was adjusted by the addition of ammonia or phosphoric acid, as appropriate. The dissolved oxygen was maintained about 20% during the whole culture by automatic variation of the stirring speed (500-900 rpm) and air flow (1-2 vvm). The end of the batch phase upon depletion of the carbon source occurred at the approximate time of 20 h as judged by a sharp increase of pH and pO₂. The feeding medium (1.5 L), consisting of 50% (v/v) glycerol, was added at an increasing flow of 5.4-8.2 mL/h/L of initial volume. In none of the recombinant clones expressing LsdA variants, inhibition of cell growth was observed during the 72 hours of culture, when compared to the growth of strain X-33 transformed with the empty pGAPZaC vector (Table 9).

Cultures were collected, centrifuged and assayed for sucrase activity in intact cells (periplasmic activity) and the culture supernatant (extracellular activity). Table 9 shows the behavior of the recombinant clones expressing the different LsdA variants. In all cases, the recombinant enzyme was retained in the periplasmic space and, to a lesser extent, released into the culture medium. The occurrence of *N*-glycosylation increased the thermal stability of the enzyme and did not cause variation in the product profile with respect to the equivalent modified variant expressed in *E. coli.*

**Table 9. Behavior of Pichia pastoris clones expressing modified LsdA variants in fed-batch fermentation**

| **LsdA variant** | **Wet biomass (g/L)** | **Periplasmic activity* (U/L)** | **Extracellular activity (U/L)** | **Total activity (U/L)** |
|---|---|---|---|---|
| Empty vector | 259 ± 12 | 0 | 0 | 0 |
| Non-mutated | 254 ± 10 | 2214 ± 127 | 404 ± 17 | 2618 |
| H142E | 247 ± 31 | 2312 ± 125 | 363 ± 44 | 2675 |
| R141K-H142S | 251 ± 21 | 2410 ± 132 | 378 ± 21 | 2788 |
| F274A | 255 ± 14 | 1578 ± 161 | 274 ± 14 | 1852 |
| N276H | 263 ± 22 | 2243 ± 134 | 413 ± 26 | 2656 |
| N276R | 250 ± 20 | 1829 ± 106 | 333 ± 22 | 2162 |
| R304K | 271 ± 18 | 2071 ± 146 | 379 ± 48 | 2450 |
| N276H-R304K | 244 ± 22 | 1893 ± 102 | 340 ± 28 | 2233 |
| N276R-R304K | 240 ± 28 | 1712 ± 114 | 332 ± 14 | 2044 |
| N276K-R304K | 261 ± 22 | 1904 ± 133 | 371 ± 31 | 2165 |

| | | | | |
|---|---|---|---|---|
| *Periplasmic activity refers to enzyme units in intact cells per liter of culture. Sucrose diffuses into the periplasmic space of the yeast. Values represent the mean of three experiments ± the standard deviation. | | | | |

### Example 10. Production of stable enzymatic preparations of LsdA variants

*Pichia pastoris* clones expressing the LsdA variants referred to in Table 9 were grown for 72 h in fed-batch fermentation using the culture medium and the operating conditions described in Example 9. After centrifugation, the culture supernatant was filtrated through a Hydrosart membrane (porosity 0.2 µm) in a Sartocon Slice 200 equipment (Sartorius, Germany). The filtrate was ten-fold concentrated and dialyzed in 100 mM sodium acetate buffer (pH 5.5) using a Hydrosart membrane (cut-off 30 kDa). After freeze drying, the activity ranged between 9,000 a 12,000 U per gram of powder. One unit (U) represents the amount of enzyme that releases 1 µmol of glucose per min in initial-rate reactions with 1.5 M sucrose in 100 mM sodium acetate buffer (pH 5.5) and 40°C. The activity of the enzyme preparations remained stable for at least 1 year of storage under refrigerated conditions (4- 10°C).

### Example 11. Establishment of a bi-enzymatic cascade process for the synthesis of a broad spectrum of FOS with complete conversion of sucrose.

The modified LsdA variants do not hydrolyze β(2,1) linked FOS. This property makes it possible their use for the conversion of the remaining sucrose in syrups containing 1-kestotriose, 1,1-kestotetraose and 1, 1,1-kestopentaose that are produced commercially using a fungal or plant fructosyltransferase. In a first-step reaction, a recombinant sucrose:sucrose 1-fructosyltransferase from the plant *Schedonorus arundinaceus* (named Sal-SSTrec) was incubated with a sucrose solution (700 g/L) at pH 5.5 in a stirred reactor at 45°C and agitation 100 rpm.. When the 1-kestotriose content reached to represent 50% (w/w) of the total carbohydrate composition, the enzyme Sal-SSTrec was inactivated by progressively increasing the temperature to 80°C over a 30 min interval. In the second step, a stable preparation of modified LsdA or the non-mutated enzyme, produced in Example 10, was added to the same reactor and incubated at 45°C until complete depletion of the remaining sucrose.

The carbohydrate composition and the profile of short-chain FOS (GF2 and GF3) in the reaction mixture was determined by the HPAEC-PAD technique, as described in Example 8. Table 10 shows the high yield of 1-kestotriose achieved in the Sal-SSTrec reaction with sucrose (step one) and confirms the inability of this enzyme to synthesize β(2,6) linked FOS. After thermal inactivation of Sal-SSTrec, the addition of a LsdA variant (step two) allowed complete sucrose depletion and diversification of the FOS spectrum. The yield and final composition of kestotrioses (GF2) and kestotetraoses (GF3) varied depending on the LsdA variant used in the second step of the bi-enzymatic process.

The two variants with amino acid substitution at the -1 subsite (H142E and R141K-H142S) hydrolyzed the sucrose remaining after the first-step reaction and did not alter the FOS composition. More interestingly, sucrose conversion by the seven LsdA variants with amino acid substitution at the +2 and/or +3 subsites (F274A, N276H, N276R, R304K, N276H-R304K, N276R-R304K and N276K-R304K) increased the contents of kestotetraoses (GF3), kestopentaoses (GF4) and eventually hexaoses (GF5). In addition to the enhanced overall FOS yield, it is relevant the predominant synthesis of 1,6-kestotetraose by the modified variants N276R y N276R-R304, as a result of the fructosyl transfer from sucrose to the OH group of carbon 6 of the terminal fructose of 1-kestotriose. Surprisingly, under the reaction conditions of step 2, the non-mutated LsdA enzyme did not form the polysaccharide levan.

**Table 10. Product profiles after the first and second reactions of the bi-enzymatic cascade system.**

| Enzyme | Short-chain FOS (% w/w) | Carbohydrate composition (% weight/weight) | | | | | | | | |
|---|---|---|---|---|---|---|---|---|---|---|
| | | G | F | S | 1K | 6K | NK | 1,1K | 1,6K | 6,6K |
| Step 1: Sal-SSTrec reaction with sucrose (700 g/L) | | | | | | | | | | |
| Sal-SSTrec | 55.7 | 19.2 | 0.3 | 23.8 | 52.9 | 0 | 0 | 2.8 | 0 | 0 |

| Step 2: reaction of a LsdA variant with sucrose remaining the FOS syrup after step 1 | | | | | | | | | | |
|---|---|---|---|---|---|---|---|---|---|---|
| Non-mutated LsdA | 60.1 | 30.2 | 2.9 | 2.8 | 30.8 | 1.1 | 0.3 | 22.5 | 3.9 | 1.5 |
| H142E | 56.2 | 31.6 | 11.5 | 0.4 | 50.8 | 0.1 | 0.1 | 5.2 | 0 | 0 |
| R141K-H142S | 56.6 | 31.4 | 11.0 | 0.4 | 51.2 | 0.1 | 0.1 | 5.4 | 0 | 0 |
| F274A | 58.7 | 30.8 | 7.8 | 1.9 | 38.1 | 0.3 | 0.3 | 11.4 | 8.3 | 0.3 |
| N276H | 59.2 | 30.9 | 8.3 | 0.7 | 47.6 | 0.3 | 0.4 | 8.6 | 2.2 | 0.1 |
| N276R | 57.9 | 31.0 | 9.6 | 0.4 | 42.1 | 1.8 | 0.4 | 3.2 | 9.6 | 0.8 |
| R304K | 60.7 | 30.9 | 6.2 | 0.9 | 35.4 | 0.4 | 1.4 | 19.8 | 3.1 | 0.6 |
| N276H-R304K | 61.2 | 30.4 | 7.0 | 0.5 | 39.0 | 0.6 | 1.2 | 12.8 | 7.2 | 0.4 |
| N276R-R304K | 57.2 | 31.2 | 10.3 | 0.4 | 38.1 | 2.1 | 0.2 | 5.7 | 10.2 | 0.9 |
| N276K-R304K | 61.6 | 30.3 | 6.8 | 0.3 | 44.2 | 1.3 | 0.4 | 6.4 | 8.8 | 0.5 |

| | | | | | | | | | | |
|---|---|---|---|---|---|---|---|---|---|---|
| Legend: G, glucose; F, fructose; S, sucrose; 1K, 1-kestotriose; 6K, 6-kestotetraose; NK, neokestotriose; 1, 1K, 1,1-kestotetraose; 1,6K, 1,6-kestotetraose; 6,6K, 6,6-kestotetraose. The short-chain FOS content refers to the sum of trisaccharides (1K, 6K and NK) and tetrasaccharides (1.1K, 1.6K and 6.6K). The modified LsdA variants with mutation in the +2 and/or +3 subsite and the non-mutated enzyme synthesized low proportions of pentasaccharides (GF4) and eventually hexasaccharides (GF5) that were not quantified in the experiment. | | | | | | | | | | |

### Example 12. Establishment of a bi-enzymatic process for the production of a glucose-free FOS syrup.

In order to eliminate the glucose by-product present in the FOS syrup produced from sucrose, the second step of the bi-enzymatic process described in Example 11 was changed. In the new process, the transformation of the products of the Sal-SSTrec reaction (step 1) was not carried out with a soluble preparation of the modified LsdA, but with whole cells of P. *pastoris* clones, which contains the modified LsdA enzyme in the periplasm. In order to make this biocatalyst reusable, the viable yeast cells were entrapped in a calcium alginate beads and stored in a sucrose solution (1.75 M) at 4°C.

Figure 1 shows the HPLC chromatograms of the intermediate and final steps of the two implemented bi-enzymatic processes. The Sal-SSTrec reaction with sucrose (700 g/L) at pH 5.5 and 45°C (step 1) produced 1-kestotriose (GF₂) and 1, 1-kestotetraose (GF₃) in a 9:1 ratio, and their sum represented 56% (w/w) of the total carbohydrate composition (Fig. 1A and Fig. 1C). The use of a soluble preparation of modified LsdA (variant R141K-H142S) in the second step of the process allowed the total hydrolysis of the remaining sucrose, without altering the sum of 1-kestotriose and 1,1-kestotetraose (Fig. 1B). Alternatively, the immobilized yeast expressing modified LsdA (R141K-H142S variant) not only hydrolyzed the remaining sucrose but also consumed all the glucose released from sucrose. The content and composition of FOS synthesized in the first step reaction remained unaltered (Fig. 1D). The absence of glucose in the final FOS syrup allows its application to be extended to prebiotic food formulations intended for diabetic patients.

## Claims

1. Modified levansucrase from *Gluconacetobacter diazotrophicus* (LsdA) comprising an amino acid sequence selected from the group of sequences composed of SEQ ID NO: 26 - SEQ ID NO: 34.

2. The levansucrase of claim 1 produced in a genetically modified host microorganism.

3. The levansucrase of claim 2 wherein the genetically modified host microorganism is the yeast *Pichia pastoris* or the bacterium *Escherichia coli.*

4. Genetically modified microorganism **characterized by** producing the modified levansucrase of any of claims 1 to 3.

5. The genetically modified microorganism of claim 4 which is the yeast *Pichia pastoris* or the bacterium *Escherichia coli.*

6. Enzymatic preparation comprising the modified levansucrase of any of claims 1 to 3.

7. The enzyme preparation of claim 6 that is a liquid or a lyophilisate.

8. Enzymatic process for the conversion of sucrose into fructooligosaccharides (FOS) comprising the use of the modified levansucrase of any of claims 1 to 3 or the genetically modified microorganism of claims 4 to 5.

9. Bi-enzymatic process for conversion of sucrose into fructooligosaccharides (FOS) **characterized by** the use of an enzyme with fructosyltransferase activity in a first step of the process and the use of the modified levansucrase of any of claims 1 to 3, the non-mutated levansucrase, or the genetically modified microorganism of claims 4 to 5 in a second step.

10. The process of claim 9 wherein the enzyme used in the first step is a sucrose:sucrose 1-fructosyltransferase (1-SST).

11. Use of the modified levansucrase of any of claims 1 to 3 or the genetically modified microorganism of claims 4 to 5 in an enzymatic process for conversion of sucrose into fructooligosaccharides (FOS).

12. The use of claim 11 wherein the process is a bi-enzymatic cascade reaction where in a first step an enzyme with fructosyltransferase activity is used and in a second step the modified levansucrase or the genetically modified microorganism is used.

13. The use of claim 12 wherein the enzyme used in the first step is a sucrose: sucrose 1-fructosyltransferase (1-SST).
